# EUROPEAN PATENT APPLICATION

(11) **EP 2 256 215 A1**
(43) Date of publication of application: **01.12.2010**
(21) Application number: 09075219.7
(22) Date of filing: 30.04.2009
(51) Int. Cl.: C12Q 1/68

(54) **Assay system using a nuclease activity of a nucleic acid polymerase**

(71) Applicant: Mergemeier, Steffen, 10243 Berlin (DE)
(72) Inventor: Mergemeier, Steffen, 10243 Berlin (DE)
(74) Representative: Boeckh, Tobias

(57) **Abstract**

The present invention is directed to a process of detecting a target nucleic acid using labelled oligonucleotides. This process uses a specific activity of a nucleic acid polymerase to cleave annealed labelled oligonucleotide from hybridized duplexes and release labelled oligonucleotide fragments for detection. This process is easily incorporated into a PCR amplification assay, such as real-time or Taqman PCR, wherein the labelled probe overlaps with the primer that anneals on the same nucleic acid strand. Thus, a triplex is formed between target, probe and primer. A polymerase having nuclease activity is used, which cleaves the labelled (eg. with FRET) probe and releases labelled fragments.

## Description

The present invention is directed to a process of detecting a target nucleic acid using labelled oligonucleotides. This process uses a specific activity of a nucleic acid polymerase to cleave annealed labelled oligonucleotide from hybridized duplexes and release labelled oligonucleotide fragments for detection. This process is easily incorporated into a PCR amplification assay.

Investigational microbiological techniques are routinely being applied to diagnostic assays. For example, Falkow et al., U.S. Pat. No. 4,358,535 disclose a method for detecting pathogens by spotting a sample e.g., blood, cells, saliva, etc. on a filter (e.g., nitrocellulose), lysing the cells, and fixing the DNA through chemical denaturation and heating. Then, labelled DNA probes are added and allowed to hybridize with the fixed sample DNA, hybridization indicating the presence of the pathogen's DNA. The sample DNA in this case may be amplified by culturing the cells or organisms in place on the filter.

A significant improvement in DNA amplification, the polymerase chain reaction (PCR) technique, is disclosed in U.S. Pat. Nos. 4,683,202, 4,683,195 and 4,800,159. In its simplest form, PCR is an in vitro method for the enzymatic synthesis of specific DNA sequences, using two oligonucleotide primers that hybridize to opposite strands and flank the region of interest in the target DNA. A repetitive series of reaction steps involving template denaturation, primer annealing, and the extension of the annealed primers by DNA polymerase results in the exponential accumulation of a specific fragment whose termini are defined by the 5' ends of the primers. The PCR method is also described in Saiki et al., (1985) Science 230:1350.

Detection methods generally employed in standard PCR techniques use a labelled probe with the amplified DNA in a hybridization assay. For example, commonly-owned copending patent application U.S. Ser. Nos. 899,344 and 178,276 to Erlich et al., disclose assay methods wherein the PCR-amplified DNA is first fixed to a filter and then a specific oligonucleotide probe is added and allowed to hybridize. Preferably, the probe is labelled, e.g., with .sup.32 P, biotin, horseradish peroxidase (HRP), etc., to allow for detection of hybridization. The reverse is also suggested, that is, the probe is instead bound to the membrane and the PCR amplified sample DNA is added.

Other means of detection include the use of fragment length polymorphism (PCR FLP), hybridization to allele-specific oligonucleotide (ASO) probes (Saiki et al., (1986) Nature 324:163), or direct sequencing via the dideoxy method (using amplified DNA rather than cloned DNA). The standard PCR technique operates (essentially) by replicating a DNA sequence positioned between two primers, providing as the major product of the reaction a DNA sequence of discrete length terminating with the primer at the 5' end of each strand. Thus, insertions and deletions between the primers result in product sequences of different lengths, which can be detected by sizing the product in PCR-FLP. In an example of ASO hybridization, the amplified DNA is fixed to a nylon filter (by, for example, UV irradiation) in a series of "dot blots", then allowed to hybridize with an oligonucleotide probe labelled with HRP under stringent conditions. After washing, tetramethylbenzidine (TMB) and hydrogen peroxide are added: HRP oxidizes the hydrogen peroxide which in turn oxidizes the TMB to a blue precipitate, indicating hybridized probe.

While the PCR technique is an extremely powerful method for amplifying nucleic acid sequences, the detection of the amplified material requires additional manipulation and subsequent handling of the PCR products to determine whether the target DNA is present. It would be desirable to decrease the number of subsequent handling steps currently required for the detection of amplified material. A "homogeneous" assay system, that is, one which generates signal while the target sequence is amplified, requiring minimal post amplification handling, would be ideal. An example for an homogeneous" assay is the real-time polymerase chain reaction, also called quantitative real time polymerase chain reaction (qRT-PCR) or kinetic polymerase chain reaction, which is a laboratory technique based on the polymerase chain reaction, which is used to amplify and simultaneously quantify a targeted nucleic acid molecule. It therefore enables both detection and quantification of a specific sequence in a DNA sample. The method follows the general principle of polymerase chain reaction. Its key feature is that the amplified DNA is quantified as it accumulates in the reaction in real time after each amplification cycle. There are two common methods of quantification: the use of fluorescent dyes that intercalate with double-stranded DNA, and fluorescence dye labelled oligonucleotide probes changing fluorescence in dependence of the formed PCR products.

The primers used in the aforementioned methods are selected to be "substantially" complementary to the different strands of each specific sequence to be amplified. The primers need not reflect the exact sequence of the template, but must be sufficiently complementary to selectively hybridize to their respective strands. Non complementary bases or longer sequences can be interspersed into the primer or located at the ends of the primer, provided the primer retains sufficient complementarity with its template strand to form a stable duplex therewith. The non complementary nucleotide sequences of the primers may include restriction enzyme sites. In addition, the labelled oligonucleotide, that fluoresce must be first annealed to its complementary nucleic acid. The technique is based on special property of nucleic acid polymerases. Nucleic acid polymerases are known to possess several activities, among them, a nuclease activity whereby the nucleic acid polymerase can cleave mononucleotides or small oligonucleotides from an oligonucleotide annealed to its larger, complementary polynucleotide. In order for cleavage to occur, a first oligonucleotide must also be annealed to the same larger polynucleotide before the nucleic acid polymerase encounters region, thereby permitting the nuclease activity to cleave and release labelled oligonucleotide fragments.

To enhance the likelihood that the labelled oligonucleotide will have annealed to its complementary nucleic acid before primer extension polymerization reaches this duplex region, or before the polymerase attaches to the first oligonucleotide in the polymerization-independent process, a variety of techniques may be employed. Short primer molecules generally require cooler temperature to form sufficiently stable hybrid complexes with the target nucleic acid. Therefore, the labelled oligonucleotide can be designed to be longer than the primer so that the labelled oligonucleotide anneals preferentially to the target at higher temperatures relative to primer annealing.

Disadvantages of the aforementioned state of the art is that the primer and the labelled oligonucleotide have to be designed that the labelled oligonucleotide anneals to the target sequence first and that the labelled oligonucleotide and the primer target a different sequence of the target nucleic acid, as an overlap of both needed to be avoided. Additionally, the synthetic oligonucleotides used in PCR experiments need to be quite long to ensure proper annealing to the target sequence, resulting in long cycle times.

It was an objective of the invention to provide a method which overcomes the problems known in the state of art and provide a process where the quantification of a known target is possible using a labelled oligonucleotide, whereby the labelled oligonucleotide and the primer are allowed to anneal to an overlapping region of the target sequence.

Surprisingly the invention solves the underlying problem by providing a process for the detection of a target nucleic acid sequence in a sample, said process comprising:
a. contacting a sample comprising single-stranded nucleic acids with an oligonucleotide containing a sequence complementary to a region of the target nucleic acid and a labelled oligonucleotide containing a sequence complementary to a second region of the same target nucleic acid sequence strand and including a part of the nucleic acid sequence region at least one nucleotide defined by the first oligonucleotide, to create a mixture of triplexes during hybridization conditions, wherein the triplexes comprise the target nucleic acid annealed to the first oligonucleotide and to the labelled oligonucleotide
b. maintaining the mixture of step (a) with a template dependent nucleic acid polymerase having a nuclease activity under conditions sufficient to permit the nuclease activity of the polymerase to cleave the annealed, labelled oligonucleotide and release labelled fragments; and
c. detecting and/or measuring the release of labelled fragments.

The sample comprises a single-stranded nucleic acid sequence to which an oligonucleotide (also called "primer") is binding to. The oligonucleotide contains a sequence, complementary to a region of the target nucleic acid sequence. In addition, a labelled oligonucleotide also binds to the target sequence, whereby the labelled oligonucleotide (also called "probe") contains a sequence complementary to a second region of the same target nucleic acid sequence strand. However, the labelled oligonucleotide includes a part or at least one nucleotide, of the nucleic acid sequence region defined by the first oligonucleotide. Thereby, a triplex of mixtures is formed during hybridization conditions, comprising the first oligonucleotide and the labelled oligonucleotide annealed to the target nucleic acid sequence. In the next step the mixture is maintained, and a template-dependent nucleic acid polymerase, which has a nuclease activity under defined conditions to permit the nuclease activity of the polymerase to cleave the annealed labelled oligonucleotide, thereby releasing labelled fragments. The labelled fragments can be measured or detected by the appropriate detection system, allowing the calculation of the amplified target.

The invention allows the annealing of the labelled oligonucleotide to a region which is partly not complementary to target sequence. The first oligonucleotide and the labelled oligonucleotide are allowed to partly overlap and the first oligonucleotide can displace the already bound labelled oligonucleotide. This saves time and work steps, as the design of the first oligonucleotide and the labelled oligonucleotide is easier. An advantage of the invention is that it allows the design short PCR-systems, that the total length of the synthetic oligonucleotides (such as both primers and labelled oligonucleotide) is less than the number of nucleotides of the target sequence. This is advantageous regarding velocity and integrity of the PCR reaction. Additionally, the consumption of consumables in the PCR reaction, such as enzyme, dNTPs or others, is reduced, which saves money and increases the efficiency of the PCR reaction. These properties are advantageous for techniques such as the multiplex PCR.

As used herein, a "sample" refers to a sample of tissue or fluid isolated from an individual or individuals, including but not limited to, for example, skin, plasma, serum, spinal fluid, lymph fluid, synovial fluid, urine, tears, blood cells, organs, tumors, and also to samples of in vitro cell culture constituents (including but not limited to conditioned medium resulting from the growth of cells in cell culture medium, recombinant cells and cell components).

As used herein, the terms "nucleic acid", "polynucleotide" and "oligonucleotide" refer to primers, probes, oligomer fragments to be detected, oligomer controls and unlabelled blocking oligomers and shall be generic to polydeoxyribonucleotides (containing 2-deoxy-D-ribose), to polyribonucleotides (containing D-ribose), and to any other type of polynucleotide which is an N glycoside of a purine or pyrimidine base, or modified purine or pyrimidine bases. There is no intended distinction in length between the term "nucleic acid", "polynucleotide" and "oligonucleotide", and these terms will be used interchangeably. These terms refer only to the primary structure of the molecule. Thus, these terms include double-and single-stranded DNA, as well as double- and single stranded RNA. The oligonucleotide is comprised of a sequence of approximately at least 6 nucleotides, preferably at least about 10-12 nucleotides, and more preferably at least about 15-20 nucleotides corresponding to a region of the designated nucleotide sequence. "Corresponding" means identical to or complementary to the designated sequence.

The oligonucleotide is not necessarily physically derived from any existing or natural sequence but may be generated in any manner, including chemical synthesis, DNA replication, reverse transcription or a combination thereof. The terms "oligonucleotide" or "nucleic acid" intend a polynucleotide of genomic DNA or RNA, cDNA, semisynthetic, or synthetic origin which, by virtue of its origin or manipulation: (1) is not associated with all or a portion of the polynucleotide with which it is associated in nature; and/or (2) is linked to a polynucleotide other than that to which it is linked in nature; and (3) is not found in nature.

Because mononucleotides are reacted to make oligonucleotides in a manner such that the 5.dbd. phosphate of one mononucleotide pentose ring is attached to the 3' oxygen of its neighbour in one direction via a phosphodiester linkage, an end of an oligonucleotide is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have a 5' and 3' ends.

The term "primer" may refer to more than one primer and refers to an oligonucleotide, whether occurring naturally, as in a purified restriction digest, or produced synthetically, which is capable of acting as a point of initiation of synthesis along a complementary strand when placed under conditions in which synthesis of a primer extension product which is complementary to a nucleic acid strand is catalyzed. Such conditions include the presence of four different deoxyribonucleoside triphosphates and a polymerization-inducing agent such as DNA polymerase or reverse transcriptase, in a suitable buffer ("buffer" includes substituents which are cofactors, or which affect pH, ionic strength, etc.), and at a suitable temperature. The primer is preferably single-stranded for maximum efficiency in amplification.

The complement of a nucleic acid sequence as used herein refers to an oligonucleotide which, when aligned with the nucleic acid sequence such that the 5' end of one sequence is paired with the 3' end of the other, is in "antiparallel association." Certain bases not commonly found in natural nucleic acids may be included in the nucleic acids of the present invention include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, percent concentration of cytosine and guanine bases in the oligonucleotide, ionic strength, and incidence of mismatched base pairs.

Stability of a nucleic acid duplex is measured by the melting temperature, or "T.sub.m." The T.sub.m of a particular nucleic acid duplex under specified conditions is the temperature at which half of the base pairs have disassociated.

As used herein, the term "target sequence" or "target nucleic acid sequence" refers to a region of the oligonucleotide which is to be either amplified, detected or both. The target sequence resides between the 5' ends of the two primer sequences used for amplification.

As used herein, the term "probe" refers to a labelled oligonucleotide which forms a duplex structure with a sequence in the target nucleic acid, due to complementarity of at least one sequence in the probe with a sequence in the target region. The term "label" as used herein refers to any atom or molecule which can be used to provide a detectable (preferably quantifiable) signal, and which can be attached to a nucleic acid or protein. Labels may provide signals detectable by fluorescence, radioactivity, colorimetric, X-ray diffraction or absorption, magnetism, enzymatic activity, and the like.

The template-specific nucleic acid polymerase has a nuclease activity traditionally associated with some DNA polymerases whereby nucleotides are removed from an oligonucleotide.

It is preferred, that at least one label is attached to an oligonucleotide. Thus, the cleaved mononucleotides or small oligonucleotides which are cleaved by the nuclease activity of the polymerase can be detected. Subsequently, any of several strategies may be employed to distinguish the uncleaved labelled oligonucleotide from the cleaved fragments thereof. In this manner, the present invention permits identification of those nucleic acid samples which contain sequences regions complementary to the first and labelled oligonucleotides.

The oligonucleotide primers and labelled oligonucleotides may be prepared by any suitable method. Methods for preparing oligonucleotides of specific sequence are known in the art, and include, for example, cloning and restriction of appropriate sequences, and direct chemical synthesis. Chemical synthesis methods may include, for example, the phosphotriester method described by Narang et al. (1979) Methods in Enzymology 68:90, the phosphodiester method disclosed by Brown et al. (1979) Methods in Enzymology 68:109, the diethylphosphoramidate method disclosed in Beaucage et al. (1981) Tetrahedron Letters 22:1859, and the solid support method disclosed in U.S. Pat. No. 4,458,066.

The oligonucleotide is labelled, as described below, by incorporating moieties detectable by spectroscopic, photochemical, biochemical, immunochemical, or chemical means. The method of linking or conjugating the label to the oligonucleotide probe depends, of course, on the type of label(s) used and the position of the label on the probe.

A variety of labels which would be appropriate for use in the invention, as well as methods for their inclusion in the probe, are known in the art and include, but are not limited to, enzymes (e.g., alkaline phosphatase and horseradish peroxidase) and enzyme substrates, radioactive atoms, fluorescent dyes, chromophores, chemiluminescent labels, electrochemiluminescent labels, such as Origin.TM. (Igen), ligands having specific binding partners, or any other labels that may interact with each other to enhance, alter, or diminish a signal. Of course, should the PCR be practiced using a Thermo Cycler instrument, the label must be able to survive the temperature cycling required in this automated process.

Among radioactive atoms, .sup.32 P is preferred. Methods for introducing sup.32 P into nucleic acids are known in the art, and include, for example, 5' labelling with a kinase, or random insertion by nick translation. Enzymes are typically detected by their activity. "Specific binding partner" refers to a protein capable of binding a ligand molecule with high specificity, as for example in the case of an antigen and a monoclonal antibody specific therefore. Other specific binding partners include biotin and avidin or streptavidin, IgG and protein A, and the numerous receptor-ligand couples known in the art. It should be understood that the above description is not meant to categorize the various labels into distinct classes, as the same label may serve in several different modes. For example, .sup.125 I may serve as a radioactive label or as an electron-dense reagent. HRP may serve as enzyme or as antigen for a monoclonal antibody. Further, one may combine various labels for desired effect. For example, one might label a probe with biotin, and detect its presence with avidin labelled with .sup.125 I, or with an anti-biotin monoclonal antibody labelled with HRP. Other permutations and possibilities will be readily apparent to those of ordinary skill in the art, and are considered as equivalents within the scope of the instant invention.

In also preferred, that the labelled oligonucleotide comprises first and second labels wherein the first label is separated from the second label by a nuclease susceptible cleavage site. In some situations it may be desirable to use two interactive labels on a single oligonucleotide with due consideration given for maintaining a nuclease susceptible cleavage site to permit the separation of the labels during oligonucleotide hydrolysis, and in other instances it may be desirable to use a single probe having two different label moieties. The detection of the hydrolyzed labelled probe can be accomplished using, for example, fluorescence polarization. This technique is able to differentiate between large and small molecules based on molecular tumbling. Large molecules (e.g., intact labelled probe) tumble in solution much more slowly than small molecules. Upon linkage of a fluorescent moiety to the molecule of interest (e.g., the 5' end of a labelled probe), this fluorescent moiety can be measured (and differentiated) based on molecular tumbling, thus differentiating between intact and digested probe. Detection may be measured directly during PCR or may be performed post PCR.

Methods for introducing oligonucleotide functionalizing reagents to introduce one or more sulfhydryl, amino or hydroxyl moieties into the oligonucleotide probe sequence, typically at the 5' terminus are described in U.S. Pat. No. 4,914,210. A 5' phosphate group can be introduced as a radioisotope by using polynucleotide kinase and [gamma.sup.32 P]ATP to provide a reporter group. Biotin can be added to the 5' end by reacting an aminothymidine residue, introduced during synthesis, with an N-hydroxysuccinimide ester of biotin.

Labels at the 3' terminus may employ polynucleotide terminal transferase to add the desired moiety, such as for example, cordycepin .sup.35 S-dATP, and biotinylated dUTP.

Oligonucleotide derivatives are also available labels. For example, etheno-dA and etheno-A are known fluorescent adenine nucleotides which can be incorporated into an oligonucleotide probe. Similarly, etheno-dC is another analogue that could be used in probe synthesis. The probes containing such nucleotide derivatives may be hydrolyzed to release much more strongly fluorescent mononucleotides by the polymerase's nuclease activity as DNA polymerase extends a primer during PCR.

It is also preferred, that the labelled oligonucleotide comprises at the 3' end a tail of non-nucleic acids or a sequence of nucleotides which is non-complementary to the target nucleic acid sequence. The labelled oligonucleotide can be designed that the 3' end does not anneal to the target region, however without affecting its specificity, as a complementary part of the labelled oligonucleotide still interacts with the target sequence. The unspecific 3' end can be used for additional applications. For example detection molecules can detect and interact with the 3' end of the labelled oligonucleotide, thereby using the sequence of the 3' tail of the labelled oligonucleotide as a specific recognition site. This is advantageous as it allows to use a labelled oligonucleotide for at least two sequential detection methods - the first one serves to detect a specific target using the complementary region of the labelled oligonucleotide and the second one specifically detects a specific non-complementary 3' end of the labelled oligonucleotide.

The process of the invention is especially suited for analysis of nucleic acid amplified by PCR. This process is an improvement over known PCR detection methods because it allows for both amplification of a target and the release of a label for detection to be accomplished in a reaction system without resort to multiple handling steps of the amplified product. Thus, in another embodiment of the invention, a polymerase chain reaction (PCR) amplification process for detecting a target nucleic acid sequence in a sample, said process comprising:
a. providing to a PCR assay containing said sample, at least one labelled oligonucleotide containing a sequence substantially complementary to a region of the target nucleic acid and including a part of the nucleic acid sequence region at least one nucleotide defined by this primer of step (b), which anneals on the same nucleic acid strand like this labelled oligonucleotide;
b. providing a set of oligonucleotide primers, wherein a first primer contains a sequence complementary to a region in one strand of the target nucleic acid sequence and primes the synthesis of a complementary DNA strand, and a second primer contains a sequence complementary to a region in a second strand of the target nucleic acid sequence and primes the synthesis of a complementary DNA strand; and wherein each oligonucleotide primer is selected to anneal to its complementary template
c. amplifying the target nucleic acid sequence employing a nucleic acid polymerase having a nuclease activity as a template-dependent polymerizing agent under conditions which are permissive for PCR cycling steps of (i) annealing of primers and labelled oligonucleotide to a template nucleic acid sequence contained within the target sequence, and (ii) a nuclease activity of said nucleic acid polymerase releases labelled fragments from the annealed triplexes comprising primer and labelled oligonucleotide and its complementary template nucleic acid sequences, thereby creating detectable labelled fragments and extending the primer wherein said nucleic acid polymerase synthesizes a primer extension product; and
d. detecting and/or measuring the release of labelled fragments to determine the presence or absence of the target sequence in the sample.

The present invention exploits a nuclease activity of the polymerase when used in conjunction with PCR. This differs from previously described PCR amplification wherein the post-PCR amplified target oligonucleotides are detected, for example, by hybridization with a probe which forms a stable duplex with that of the target sequence under stringent to moderately stringent hybridization and wash conditions. In contrast to those known detection methods used in post-PCR amplifications, the present invention permits the detection of the target nucleic acid sequences during amplification of this target nucleic acid. In the present invention, a labelled oligonucleotide is added concomitantly with the primer at the start of PCR, and the signal generated from hydrolysis of the labelled nucleotide(s) of the probe provides a means for detection of the target sequence during its amplification.

A special method of the PCR is the real time PCR (qRT-PCR), as it allows the quantification of the amplified target in real-time. Polymerase Chain Reaction is abbreviated as "PCR". The term "real-time PCR" is intended to mean any amplification technique which makes it possible to monitor the progress of an ongoing amplification reaction as it occurs (i.e. in real time). Data is therefore collected during the exponential phase of the PCR reaction, rather than at the end point as in conventional PCR. Measuring the kinetics of the reaction in the early phases of PCR provides distinct advantages over traditional PCR detection. In real-time PCR, reactions are characterized by the point in time during cycling when amplification of a target is first detected rather than the amount of target accumulated after a fixed number of cycles. The higher the starting copy number of the nucleic acid target, the sooner a significant increase in fluorescence is observed. Traditional PCR methods use separation methods, such as agarose gels, for detection of PCR amplification at the final phase of or end-point of the PCR reaction. For qRT-PCR no post-PCR processing of the unknown DNA sample is necessary as the quantification occurs in real-time during the reaction. Furthermore, an increase in reporter fluorescent signal is directly proportional to the number of amplicons generated. The qRT-PCR can be applied to applications such as viral quantification, quantification of gene expression, array verification, drug therapy efficacy, DNA damage measurement, quality control and assay validation, pathogen detection and genotyping.

In either process described herein, a sample is provided which is suspected of containing the particular oligonucleotide sequence of interest, the "target nucleic acid". The target nucleic acid contained in the sample may be first reverse transcribed into cDNA, if necessary, and then denatured, using any suitable denaturing method, including physical, chemical, or enzymatic means, which are known to those of skill in the art. A preferred physical means for strand separation involves heating the nucleic acid until it is completely (>99%) denatured. Typical heat denaturation involves temperatures ranging from about 80.degree. C. to about 105.degree. C., for times ranging from about 2 seconds to 10 minutes. As an alternative to denaturation, the target nucleic acid may exist in a single-stranded form in the sample, such as, for example, single stranded RNA or DNA viruses.

The denatured nucleic acid strands are then incubated with preselected oligonucleotide primers and labelled oligonucleotide (also referred to herein as "probe") under hybridization conditions, conditions which enable the binding of the primers and probes to the single nucleic acid strands. As known in the art, the primers are selected so that their relative positions along a duplex sequence are such that an extension product synthesized from one primer, when the extension produced is separated from its template (complement), serves as a template for the extension of the other primer to yield a replicate chain of defined length.

Because the complementary strands are longer than either the probe or primer, the strands have more points of contact and thus a greater chance of finding each other over any given period of time. A high molar excess of probe, plus the primer, helps tip the balance toward primer and probe annealing rather than template reannealing.

The primer must be sufficiently long to prime the synthesis of extension products in the presence of the agent for polymerization. The exact length and composition of the primer will depend on many factors, including temperature of the annealing reaction, source and composition of the primer, proximity of the probe annealing site to the primer annealing site, and ratio of primer:probe concentration. For example, depending on the complexity of the target sequence, the oligonucleotide primer typically contains about 15-30 nucleotides, although it may contain more or fewer nucleotides. The primers must be sufficiently complementary to selectively anneal to their respective strands and form stable duplexes.

The primers used herein are selected to be "substantially" complementary to the different strands of each specific sequence to be amplified. The primers need not reflect the exact sequence of the template, but must be sufficiently complementary to selectively hybridize to their respective strands. Non complementary bases or longer sequences can be interspersed into the primer or located at the ends of the primer, provided the primer retains sufficient complementarity with its template strand to form a stable duplex therewith. The non complementary nucleotide sequences of the primers may include restriction enzyme sites.

The composition of the labelled oligonucleotide can be designed to favour nuclease activity over strand displacement (mono- and dinucleotide fragments over oligonucleotides) by means of choice of sequences which are GC rich or which avoid sequential A's and T's and by choice of label position in the probe. It has been determined that in the presence of AT rich sequences in the 5' complementary probe region, cleavage occurs after the approximately fourth, fifth or sixth nucleotide. However, in a GC-rich 5' complementary probe region, cleavage generally occurs after the first or second nucleotide. Alternatively, the incorporation of modified phosphodiester linkages (e.g., methyl phosphorylthioate or methylphosphonates) in the labelled probe during chemical synthesis (Noble et al., (1984) Nuc Acids Res 12:3387-3403; lyer et al., (1990) J Am Chem Soc 112:1253-1254) may be used to prevent cleavage at a selected site. Depending on the length of the probe, the composition of its 5' complementary region, and the position of the label, one can design a probe to preferentially favour the generation of short or long labelled probe fragments for use in the practice of the invention.

Template-dependent extension of the oligonucleotide primer(s) is catalyzed by a polymerizing agent in the presence of adequate amounts of the four deoxyribonucleoside triphosphates (dATP, dGTP, dCTP, and dTTP) or anagoges as discussed above, in a reaction medium which is comprised of the appropriate salts, metal cations, and pH buffering system. Suitable polymerizing agents are enzymes known to catalyze primer and template-dependent DNA synthesis and possess nuclease activity.

It is preferred, that the nucleic acid polymerase is a thermostable enzyme. Temperature stable polymerases are preferred in this process because the preferred way of denaturing the double stranded extension products is by exposing them to a high temperature (about 95.degree. C.) during the PCR cycle. For example, U.S. Pat. No. 4,889,818 discloses a representative thermostable enzyme isolated from Thermus aquaticus. Additional representative temperature stable polymerases include, e.g., polymerases extracted from the thermostable bacteria Thermus flavus, Thermus ruber, Thermus thermophilus, Bacillus stearothermophilus (which has a somewhat lower temperature optimum than the others listed), Thermus lacteus, Thermus rubens, Thermotoga maritima, Thermococcus littoralis, and Methanothermus fervidus.

Thermostable DNA polymerases include, for example, E. coli DNA polymerase I, DNA polymerases from the Thermus species, such as Thermus thermophilus (Tth) DNA polymerase, Bacillus stearothermophilus DNA polymerase, Thermococcus littoralis DNA polymerase, and Thermus aquaticus (Taq) DNA polymerase. The reaction conditions for catalyzing DNA synthesis with these DNA polymerases are well known in the art. To be useful in the present invention, the polymerizing agent must efficiently cleave the oligonucleotide and release labelled fragments so that the signal is directly or indirectly generated.

It is also preferred, that the 3' terminus of the labelled oligonucleotide has a blocked 3' terminus to prevent extension by the nucleic acid polymerase, and thereby prohibiting incorporation of the probe into a primer extension product. Blocking may be achieved by using non-complementary bases or by adding a chemical moiety such as biotin or even a phosphate group to the 3' hydroxyl of the last nucleotide, which may, depending upon the selected moiety, may serve a dual purpose by also acting as a label.

It is preferred, that the labelled oligonucleotide further comprises at the 3'end a tail of non-nucleic acids or a sequence of nucleotides which is substantially non-complementary to the target nucleic acid sequence. The labelled oligonucleotide needs not reflect the exact sequence of the template, but must be sufficiently complementary to selectively hybridize to the respective strands. Non complementary bases or longer sequences can be interspersed into the labelled oligonucleotide, provided the labelled oligonucleotide retains sufficient complementarity with its template strand to form a stable binding therewith. The 3' end of the labelled oligonucleotide, which is non-complementary to the target sequence allows the combination of a PCR detection method with an additional detection method. Specific detection molecules can recognize and interact with the non-complementary 3' end of the labelled oligonucleotide after its binding to the target sequence. The preferred embodiment therefore provides the possibility to first detect the target sequence and then to be detected by an additional detection method. It opens a new field as several complex detections can be combined using just one synthetic labelled oligonucleotide.

In addition, the design of the primer and the labelled oligonucleotide are much easier, as both the primer and the labelled oligonucleotide can be designed to be shorter. Only a few bases need to be complementary to the target sequence, making the hybridization process more efficient and reducing the costs.

It is also preferred, that the labelled oligonucleotide comprises first and second labels wherein the first label is separated from the second label by a nuclease susceptible cleavage site. In some situations it may be desirable to use two interactive labels on a single oligonucleotide with due consideration given for maintaining a nuclease susceptible cleavage site to permit the separation of the labels during oligonucleotide hydrolysis, and in other instances it may be desirable to use a single probe having two different label moieties. In a preferred embodiment, detection of the hydrolyzed labelled probe can be accomplished using, for example, fluorescence polarization. This technique is able to differentiate between large and small molecules based on molecular tumbling. Large molecules (e.g., intact labelled probe) tumble in solution much more slowly than small molecules. Upon linkage of a fluorescent moiety to the molecule of interest (e.g., the 5' end of a labelled probe), this fluorescent moiety can be measured (and differentiated) based on molecular tumbling, thus differentiating between intact and digested probe. Detection may be measured directly during PCR or may be performed post PCR.

It is preferred, that the label is attached to a part of the tail of non-nucleic acids or substantially non-complementary nucleotides. The label can be attached to the oligonucleotide sequence, which is complementary to the target sequence, but may also be attached to a part of the tail of non-nucleic acids or substantially non-complementary nucleotides. This region is not binding to the target sequence and offers a possibility to easily detect the label, without interfering with the complementary region of the labelled oligonucleotide. In addition, the combination of more than one detection methods is possible. The oligonucleotide specifically detects a target sequence and binds to it with its complementary region. Therefore the preferred embodiment allows first the detection of the target sequence by the labelled oligonucleotide and second the detection of the labelled oligonucleotide, or more precisely the label of the non-complementary region by an additional detection method. The second detection method includes methods such as microarray, Multiplex PCR, PCR-ELISA, T.sub.m analysis or MLPA. The preferred embodiment therefore increases the efficiency and results in a better yield.

It is also preferred, that the labelled oligonucleotide comprises a pair of interactive signal-generating labels effectively positioned on the oligonucleotide to quench the generation of detectable signal, said labels being separated by a site within the oligonucleotide susceptible to nuclease cleavage, thereby allowing a nuclease activity of the nucleic acid polymerase to separate the first interactive signal generating label from the second interactive signal generating label by cleaving at the susceptible site thereby yielding a detectable signal. The oligonucleotide is designed, that the enzyme is able to access the labelled oligonucleotide and remove nucleotides from the oligonucleotide without being sterically impaired by the labels. This ensures a release of the label and subsequently proper quantification of the amplification.

It is preferred, that said first label is a fluorescence dye and said second label is a quencher compound which interacts therewith.

The terms "fluorescence dye" refers to compounds with a fluorescent emission maximum between about 400 and 900 nm. These compounds include, with their emission maxima in nm in brackets, Cy2 (506), GFP (Red Shifted) (507), YO-PRO-1 (509), YOYO.TM.-1 (509), Calcein (517), FITC (518), FluorX (519), Alexa (520), Rhodamine 110 (520), 5-FAM (522), Oregon Green 500 (522), Oregon Green 488 (524), RiboGreen (525), Rhodamine Green (527), Rhodamine 123 (529), Magnesium Green (531), Calcium Green (533), TO-PRO.-1 (533), TOTO -1 (533), JOE (548), BODIPY 530/550 (550), Dil (565), BODIPY (568), BODIPY 558/568 (568), BODIPY 564/570 (570), Cy3 (570), Alexa 546 (570), TRITC (572), Magnesium Orange (575), Phycoerythrin R&B (575), Rhodamine Phalloidin (575), Calcium Orange (576), Pyronin Y (580), Rhodamine B (580), TAMRA (582), Rhodamine Red (590), Cy3.5 (596), ROX (608), Calcium Crimson (615), Alexa 594 (615), Texas Red (615), Nile Red (628), YO-PRO-3 (631), YOYO-3 (631), R-phycocyanin (642), C-Phycocyanin (648), TO-PRO-3 (660), TOTO-3 (660), DiD DilC(5) (665), Cy5 (670), Thiadicarbocyanine (671), Cy5.5 (694) or 6-FAM (517).

A close proximity of the fluorescence dye and the quencher compound prevents detection of the fluorescence of the fluorescence dye. Breakdown of the labelled oligonucleotide by the DNA polymerase breaks the fluorescence dye-quencher compound proximity and thus allows the unquenched emission of fluorescence, which then can be detected. The quencher functions as an acceptor which absorbs the emission of the fluorescence dye (Förster resonance energy transfer - FRET) and prevents the detection of the fluorescence dye. Also compatible with the invention is the technique based on FRET, named "Dye-labelled oligonucleotide ligation" (DOL). Both methods are already described in e.g. [Chen, X., Livak, K.J. & Kwok, P.Y. Genome Res. 8, 549-556 (1998), Landegren, U., Nilsson, M. & Kwok, P.Y. Genome Res. 8, 769-776 (1998). US 6,027,889; US 6,268,148] and various styles of application can be found in [Holland, P.M., Abramson, R.D., Watson, R. & Gelfand, D.H. Proc.Natl.Acad.Sci.U.S.A. 88, 7276-7280 (1991), Didenko, V.V. Biotechniques 31, 1106-1 (2001); US 5,210,015; US 5,487,972; EP 0 919 565 A2; WO 92/02638].

The invention is not limited to FRET- or DOL- based methods and can be applied to non FRET-based detection systems, such as "Molecular beacons" [Kwiatkowski, R.W., Lyamichev, V., de Arruda, M. & Neri, B. Mol.Diagn. 4, 353-364 (1999), US 5,989,823], or "Self priming probes" (US 5,866,336), or LightUp-method (EP 1 357 185 A1, US 6,461,871 B1) and the PCR-independent Invader-Assay [Cooksey, R.C., Holloway, B.P., Oldenburg, M.C., Listenbee, S. & Miller, C.W. Antimicrob. Agents Chemother. 44, 1296-1301 (2000)].

The advantage of the aforementioned techniques is, that only the emission of the fluorescence dye can be detected when the quencher compound and fluorescence dye are dissociated. Binding of the labelled oligonucleotide to the target or to an unspecific sequence or the labelled oligonucleotide alone does not generate a signal. Only the specific binding of the labelled oligonucleotide to the target and the amplification of the target by DNA polymerase generates a signal, as the nuclease activity of the enzyme removes the quencher compound from the proximity of the fluorescence dye. Quenchers can be fluorescent e.g. TAMRA (tetramethylrhodamine) or non-fluorescent e.g. derivatives of 4-(dimethylamino)azobenzene (Dabcyl), Qxl quenchers, lowa black FQ quenchers, lowa black RQ, or black hole quenchers (BHQ). Non-fluorescent quenchers are also referred to as dark quenchers, which have two advantages over fluorescent quenchers: the absence of background fluorescence and the preclusion of detection of acceptor fluorescence at the reporter fluorescence emission channel.

It is also preferred, that the label provides a signal proportional to the number of target nucleic acid sequences amplified. The release of the label can be detected and quantified, thereby allowing an accurate calculation of the nucleic acid sequences amplified. Additionally, when two labels are used, such as a fluorescence dye and a quencher, and the proximity of the fluorescence dye and the quencher compound is broken by the nuclease activity of the DNA polymerase, an increase in the product targeted by the probe at each PCR cycle causes a proportional increase in fluorescence due to the breakdown of the probe and release of the fluorescence dye. Using a quencher and a fluorescence dye is the most accurate and most reliable methods, as it directly proportional to amplification and therefore presents an achievement forwards the development.

The labels may be attached to the oligonucleotide directly or indirectly by a variety of techniques. It is preferred, that the label of the labelled oligonucleotide is attached through a spacer arm of sufficient length to permit a nuclease activity of the nucleic acid polymerase to release fragments. Depending on the precise type of label used, the label might be located at the 5' or 3' end of the probe, located internally in the probe's nucleotide sequence, or attached to spacer arms of various sizes and compositions to facilitate signal interactions. Using a spacer ensures, that the nuclease activity of the polymerase can remove the nucleotides from the oligonucleotide and is not inhibited by the label, making the constant and reliable quantification of the product possible. In addition, using a spacer allows to design the oligonucleotide independent form the label and attach the label in a separate process. Using commercially available phosphoramidite reagents, one can produce oligomers containing functional groups (e.g., thiols or primary amines) at either terminus via an appropriately protected phosphoramidite, and can label them using protocols described in, for example, PCR Protocols: A Guide to Methods and Applications, ed. by Innis et al., Academic Press, Inc., 1990.

It is also preferred, that the T.sub.m of the labeld oligonucleotide is higher than the T.sub.m of the oligonucleotide primer which anneals on the same nucleic acid strand. One can also use primers and labelled oligonucleotides having differential thermal stability. For example, the nucleotide composition of the labelled oligonucleotide can be chosen to have greater G/C content and, consequently, greater thermal stability than the primer. The thermocycling parameters can also be varied to take advantage of the differential thermal stability of the labelled oligonucleotide and primer. For example, following the denaturation step in thermocycling, an intermediate temperature may be introduced which is permissible for labelled oligonucleotide binding but not primer binding, and then the temperature is further reduced to permit primer annealing and extension.

## Claims

1. A process for the detection of a target nucleic acid sequence in a sample, said process comprising:
a. contacting a sample comprising single-stranded nucleic acids with an oligonucleotide containing a sequence complementary to a region of the target nucleic acid and a labelled oligonucleotide containing a sequence complementary to a second region of the same target nucleic acid sequence strand and including a part of the nucleic acid sequence region at least one nucleotide defined by the first oligonucleotide, to create a mixture of triplexes during hybridization conditions, wherein the triplexes comprise the target nucleic acid annealed to the first oligonucleotide and to the labelled oligonucleotide
b. maintaining the mixture of step (a) with a template-dependent nucleic acid polymerase having a nuclease activity under conditions sufficient to permit the nuclease activity of the polymerase to cleave the annealed, labelled oligonucleotide and release labelled fragments; and
c. detecting and/or measuring the release of labelled fragments.

2. The process of claim 1 wherein said labelled oligonucleotide comprises at least one label.

3. The process of claim 1 or 2 wherein the labelled oligonucleotide comprises first and second labels wherein the first label is separated from the second label by a nuclease susceptible cleavage site.

4. The process of at least one of the preceding claims wherein the labelled oligonucleotide further comprises at the 3' end a tail of non-nucleic acids or a sequence of nucleotides which is non-complementary to the target nucleic acid sequence.

5. A polymerase chain reaction (PCR) amplification process for detecting a target nucleic acid sequence in a sample, said process comprising:
a. providing to a PCR assay containing said sample, at least one labelled oligonucleotide containing a sequence substantially complementary to a region of the target nucleic acid and including a part of the nucleic acid sequence region at least one nucleotide defined by this primer of step (b), which anneals on the same nucleic acid strand like this labelled oligonucleotide;
b. providing a set of oligonucleotide primers, wherein a first primer contains a sequence complementary to a region in one strand of the target nucleic acid sequence and primes the synthesis of a complementary DNA strand, and a second primer contains a sequence complementary to a region in a second strand of the target nucleic acid sequence and primes the synthesis of a complementary DNA strand; and wherein each oligonucleotide primer is selected to anneal to its complementary template
c. amplifying the target nucleic acid sequence employing a nucleic acid polymerase having a nuclease activity as a template-dependent polymerizing agent under conditions which are permissive for PCR cycling steps of (i) annealing of primers and labelled oligonucleotide to a template nucleic acid sequence contained within the target sequence, and (ii) a nuclease activity of said nucleic acid polymerase releases labelled fragments from the annealed triplexes comprising primer and labelled oligonucleotide and its complementary template nucleic acid sequences, thereby creating detectable labelled fragments and extending the primer wherein said nucleic acid polymerase synthesizes a primer extension product; and
d. detecting and/or measuring the release of labelled fragments to determine the presence or absence of the target sequence in the sample.

6. The PCR process of claim 5 wherein said nucleic acid polymerase is a thermostable enzyme.

7. The PCR process of claim 5 or 6 wherein said labelled oligonucleotide has a blocked 3' terminus to prevent extension by the nucleic acid polymerase.

8. The PCR process of at least one of the preceding claims wherein the labelled oligonucleotide further comprises at the 3' end a tail of non-nucleic acids or a sequence of nucleotides which is substantially non-complementary to the target nucleic acid sequence.

9. The PCR process of at least one of the preceding claims wherein the labelled oligonucleotide comprises first and second labels wherein the first label is separated from the second label by a nuclease susceptible cleavage site.

10. The PCR process of at least one of the preceding claims wherein the label is attached to a part of the tail of non-nucleic acids or substantially non-complementary nucleotides.

11. The PCR process of at least one of the preceding claims wherein the label provides a signal proportional to the number of target nucleic acid sequences amplified.

12. The PCR process of at least one of the preceding claims wherein the labelled oligonucleotide comprises a pair of interactive signal-generating labels effectively positioned on the oligonucleotide to quench the generation of detectable signal, said labels being separated by a site within the oligonucleotide susceptible to nuclease cleavage, thereby allowing a nuclease activity of the nucleic acid polymerase to separate the first interactive signal generating label from the second interactive signal generating label by cleaving at the susceptible site thereby yielding a detectable signal.

13. The PCR process of at least one of the preceding claims wherein said first label is a fluorescence dye and said second label is a quencher compound which interacts therewith.

14. The PCR process of at least one of the preceding claims wherein the label of said oligonucleotide is attached through a spacer arm of sufficient length to permit a nuclease activity of the nucleic acid polymerase to release labelled fragments.

15. The PCR process of at least one of the preceding claims wherein the T.sub.m of the labelled oligonucleotide is higher than the T.sub.m of the oligonucleotide primer which anneals on the same nucleic acid strand.
